(19) Europäisches Patentamt
European Patent Office
Office européen des brevets

(11) Numéro de publication: **0 526 313 B1**

# (12) FASCICULE DE BREVET EUROPEEN

(45) Date de publication de fascicule du brevet: **19.04.95**

(21) Numéro de dépôt: **92402131.4**

(22) Date de dépôt: **23.07.92**

(51) Int. Cl.[6]: **C07C 275/30**, C07C 275/24, C07C 275/54, C07C 335/16, C07C 335/18, C07D 213/75, A61K 31/17

## (54) Nouveaux dérivés de l'urée, leur préparation et leur application en thérapeutique.

(30) Priorité: **25.07.91 FR 9109446**

(43) Date de publication de la demande:
**03.02.93 Bulletin 93/05**

(45) Mention de la délivrance du brevet:
**19.04.95 Bulletin 95/16**

(84) Etats contractants désignés:
**AT BE CH DE DK ES FR GB GR IT LI LU MC NL PT SE**

(56) Documents cités:
**EP-A- 0 007 184**
**US-A- 3 049 562**
**US-A- 3 061 640**
**US-A- 4 297 373**

(73) Titulaire: **PIERRE FABRE MEDICAMENT**
**45, Place Abel Gance**
**F-92100 Boulogne (FR)**

(72) Inventeur: **Vidaluc, Jean-Louis**
**Jean Petit**
**F-81100 Castres (FR)**
Inventeur: **Bigg, Dennis**
**122 avenue de Lavaur**
**F-81100 Castres (FR)**

(74) Mandataire: **Ahner, Francis et al**
**CABINET REGIMBEAU**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention, réalisée au Centre de Recherche Pierre Fabre, a pour objet de nouveaux dérivés de l'urée, leur préparation et leur application en thérapeutique.

Les composés de l'invention répondent à la formule générale 1 :

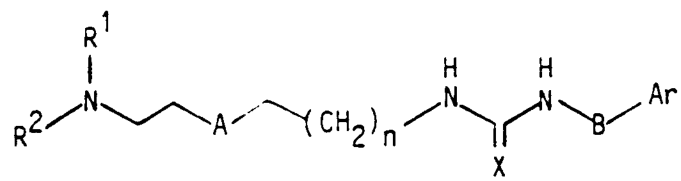

1

dans laquelle :

$R^1$ représente un groupe alkyle en $C_1$-$C_4$ ;

$R^2$ représente :

- un groupe cycloalkyle en $C_5$-$C_7$
- un groupe cycloalkylméthyle où le radical cycloalkyle comporte de 5 à 7 atomes de carbone
- un groupe benzyle
- un groupe benzyle où le cycle aromatique porte un groupe alkyle en $C_1$-$C_4$, un groupe alkoxy en $C_1$-$C_4$, un atome d'halogène ou un groupe nitro ;

A représente un atome d'oxygène ou un radical méthylène ;

n représente 1 ou 2 ;

X représente un atome d'oxygène ou de soufre ;

B représente une liaison directe, un radical méthylène ou un radical carbonyle ;

Ar représente :

- un groupe pyridyle
- un groupe phényle de formule :

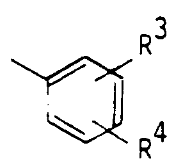

où $R_3$ et $R_4$, indépendamment l'un de l'autre, représentent un atome d'hydrogène, un atome d'halogène, un groupe nitro, un groupe alkyle en $C_1$-$C_4$, un groupe alkoxy en $C_1$-$C_4$, un groupe phényle, ou un groupe trifluorométhoxy ;

- un groupe oxofluorényle de formule :

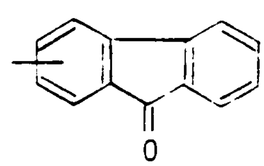

- un groupe dioxoanthracényle de formule :

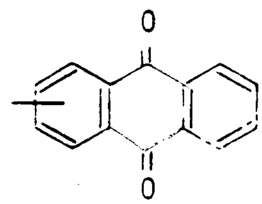

- un groupe naphthyle.

L'invention couvre également les sels des composés de formule générale 1 avec des acides minéraux ou organiques pharmaceutiquement acceptables. Parmi les acides pharmaceutiquement acceptables, on peut citer, à titre d'exemple, l'acide chlorhydrique ou l'acide fumarique.

Les composés de formule générale 1 de l'invention peuvent être préparés selon le schéma de réaction suivant :

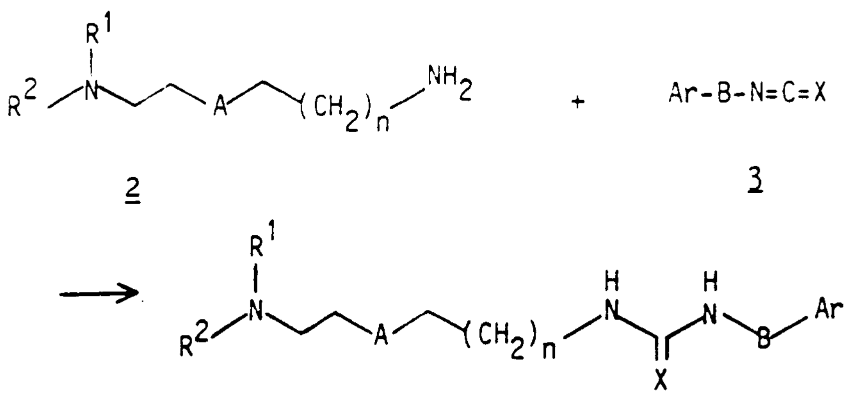

2

3

1

où $R^1$, $R^2$, A, n, B, X et Ar sont définis comme ci-dessus.

Les amines de départ 2 et les hétérocumulènes de formule 3 peuvent être préparés selon des méthodes classiques.

Lorsque X représente un atome de soufre, la réaction d'une amine de formule générale 2 avec un composé de formule générale 3 peut être effectuée à une température allant de la température ambiante à la température de reflux du solvant.

Le solvant employé peut être, à titre d'exemple, un solvant chloré tel que le dichlorométhane ou le dichloroéthane, un solvant cétonique tel que l'acétone, un solvant alcoolique, par exemple l'éthanol ou un éther tel que le tétrahydrofurane.

Lorsque X représente un atome d'oxygène, la réaction entre une amine de formule générale 3 et un isocyanate de formule générale 3 peut être effectuée à une température comprise entre 0 °C et 35 °C au sein d'un solvant aprotique. Le solvant employé peut être, à titre d'exemple, l'acétone, le toluène, un solvant chloré tel que le dichlorométhane ou le dichloroéthane, ou un éther tel que le tétrahydrofurane.

Les sels des composés de l'invention peuvent être obtenus à partir d'une solution de la base libre par l'addition d'une solution d'un acide, tel que l'acide fumarique, l'acide maléique, ou l'acide chlorhydrique, dans un solvant approprié tel que l'éthanol. La précipitation du sel peut être facilitée par l'addition d'un solvant tel que l'éther ou l'acétate d'éthyle.

Les exemples suivants illustrent l'invention sans toutefois en limiter la portée.

Les analyses et les spectres IR et RMN confirment la structure des composés obtenus selon l'invention.

**Exemple 1**

**Benzyl-1 {[(N-cyclohexyl-N-éthyl)amino-2 éthoxy]-2 éthyl}-3 urée : composé n°2 ; $R^1$ = Et, $R^2$ = Cyclohexyle, A = O, n = 1, X = O, B = $CH_2$, Ar = $C_6H_5$.**

On ajoute 0,7 ml d'isocyanate de benzyle à 10 ml de dichlorométhane et on refroidit la solution à l'aide d'un bain de glace. On ajoute une solution de 1,2 g de [(N-cyclohexyl-N-éthyl)amino-2 éthoxy]-2 éthylamine dans 5 ml de dichlorométhane avec agitation.
On laisse le mélange réactionnel revenir à la température ambiante et, après 18 heures on évapore le solvant. Le résidu huileux marron est purifié par chromatographie sur gel de silice éluée par un mélange acétate d'éthyle/méthanol (50/50). On obtient une huile qui cristallise dans l'hexane. Après filtration et séchage sous vide, on obtient 1,0 g du composé 2 sous forme de cristaux beige pâle.
PF° : 66-67°C.

**Exemple 2**

**Benzoyl-1 [(N-cyclohexylméthyl-N-méthyl)amino-5 penty]-3 thiourée : composé n°5 ; $R^1$ = Me, $R^2$ = Cyclohexylméthyl, A = $CH_2$, n = 1, X = S, B = CO, Ar =$C_6H_5$.**

On ajoute 0,25 ml de benzoyl isothiocyanate à une solution de 0,4 g de [(N-cyclohexylméthyl-N-méthyl)amino-5 pentylamine dans 10 ml de dichlorométhane. Après une nuit sous agitation à la température ambiante on évapore le solvant sous pression réduite. L'huile orangée obtenue est purifiée par chromatographie sur gel de silice éluée par le chloroforme/méthanol (95/5). On récupère après évaporation 0,6 g d'une huile orangée que l'on traite avec une solution éthanolique d'acide fumarique et de l'éther pour obtenir 0,55 g du fumarate du composé 5 sous forme de cristaux blancs.
PF° : 103-4°C.

**Exemple 3**

**{[(N-benzyl-N-méthyl)amino-2 éthoxy]-2 éthyl}-1 $\alpha$-naphtyl thiourée : composé n°7 ; $R^1$ = Me, $R^2$ = $C_6H_5CH_2$, A = O, n = 1, X = S, B = liaison directe, Ar = $\alpha$-naphtyle.**

On ajoute une solution de 1,85 g d'isothiocyanate de naphtyle dans 30 ml de dichlorométhane à 2,08 g de [(N-benzyl-N-méthyl)amino-2 éthoxy]-2 éthylamine et on agite le milieu à température ambiante pendant 16 heures. Le solvant est évaporé et l'huile jaune pâle obtenue cristallise. Le solide est repris par l'éther isopropylique et après filtration et séchage, on obtient 2,85 g du composé 6 sous forme de cristaux blancs.
PF° : 116-117°C.

**Exemple 4**

**Benzoyl-1 {[(N-benzyl-N-méthyl)amino-2 éthoxy]-3 propyl} thiourée : composé n°8 ; $R^1$ = Me, $R^2$ = $C_6H_5CH_2$, A = O, n = 2, X = S, B = CO, Ar = $C_6H_5$.**

On ajoute 1,4 ml de benzoyl isothiocyanate à une solution de 2,22 g de [(N-benzyl-N-méthyl)amino-2 éthoxy]-3 propylamine dans 20 ml d'éthanol absolu. On porte à reflux pendant 1 heure et on évapore le solvant sous pression réduite. Le résidu huileux obtenu est purifié par chromatographie sur gel de silice éluée par le chloroforme. On obtient 1,1 g d'une huile jaune pâle que l'on traite avec une solution éthanolique d'acide fumarique et de l'éther pour donner 0,7 g du fumarate du composé 8 sous forme de cristaux blancs.
PF° : 97-100°C.

Le tableau 1 ci-après résume les principaux produits synthétisés qui illustrent l'invention sans toutefois en limiter la portée.

## Tableau 1

| Comp n° | $R^1$ | $R^2$ | A | n | X | B | Ar | Sel/Base | PF(°C) |
|---|---|---|---|---|---|---|---|---|---|
| 1 | Me | $C_6H_5CH_2$ | O | 1 | O | l.d.* | $4-NO_2.C_6H_4$ | HCl | 176-7 |
| 2 | Et | $c.C_6H_{11}$ | O | 1 | O | $CH_2$ | $C_6H_5$ | Base | 66-7 |
| 3** | Me | $C_6H_5CH_2$ | O | 1 | O | CO | $C_6H_5$ | Base | Huile |
| 4 | Me | $C_6H_5CH_2$ | $CH_2$ | 1 | S | CO | $C_6H_5$ | 1/2(Fumarate) | 140-2 |
| 5 | Me | $c.C_6H_{11}CH_2$ | $CH_2$ | 1 | S | CO | $C_6H_5$ | Fumarate | 103-4 |
| 6 | Me | $C_6H_5CH_2$ | O | 1 | S | l.d. | $4-Cl.C_6H_4$ | HCl | 133-5 |
| 7 | Me | $C_6H_5CH_2$ | O | 1 | S | l.d. | α-naphthyle | Base | 116-7 |
| 8 | Me | $C_6H_5CH_2$ | O | 2 | S | CO | $C_6H_5$ | Fumarate | 97-100 |
| 9 | Me | $C_6H_5CH_2$ | O | 1 | S | CO | $C_6H_5$ | HCl | 116-18 |
| 10 | Me | $C_6H_5CH_2$ | O | 1 | S | CO | $4-Me.C_6H_4$ | Fumarate | 152-3 |
| 11 | Me | $C_6H_5CH_2$ | O | 1 | S | CO | $4-MeO.C_6H_4$ | HCl | 140-2 |
| 12 | Me | $C_6H_5CH_2$ | O | 1 | S | CO | $4-Cl.C_6H_4$ | Fumarate | 149-51 |
| 13 | Me | $C_6H_5CH_2$ | O | 1 | S | CO | $3-NO_2.C_6H_4$ | Fumarate | 94-6 |
| 14 | Me | $C_6H_5CH_2$ | O | 1 | S | CO | $3,4-Cl_2.C_6H_3$ | Fumarate | 119-21 |
| 15 | Me | $C_6H_5CH_2$ | O | 1 | S | CO | $4-Ph.C_6H_4$ | HCl | 130-2 |
| 16 | Me | $C_6H_5CH_2$ | O | 1 | S | CO | β-naphthyle | HCl | 202-4 |
| 17 | Me | $C_6H_5CH_2$ | O | 1 | S | CO | 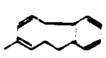 | HCl | 205-10 |
| 18 | Me | $C_6H_5CH_2$ | O | 1 | S | CO | 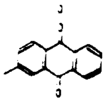 | Base | 113-5 |
| 19 | Me | $c.C_6H_{11}$ | O | 1 | S | CO | $C_6H_5$ | 1/2(Fumarate) | 140-2 |
| 20 | Me | $3-NO_2.C_6H_4CH_2$ | O | 1 | S | l.d. | 3-pyridyle | Fumarate | 70-90 |
| 21 | Me | $C_6H_5CH_2$ | O | 1 | S | l.d. | $4-CF_3O.C_6H_4$ | HCl | 142-3 |
| 22 | Me | $3-NO_2.C_6H_4CH_2$ | O | 1 | S | l.d. | $3,4-(MeO)_2.C_6H_3$ | HCl | 118-25 |

* liaison directe

** IR(KBr) : 1680 $cm^{-1}$. RMN($CDCl_3$/TMS) : δ, (multiplicité, nombre de protons) : 2.27(s,3H) ; 2.66(t,2H) ; 3.62(m,8H) ; 7.36(m,5H) ; 7.50(m,3H) ; 8.02(m,2H) ; 9.06(t,1H) ; 10.13(s,1H).

Les composés de l'invention ont été soumis à des essais pharmacologiques qui ont montré leur intérêt en tant qu'inhibiteurs de l'acétylcholinestérase.

A cet effet, les composés ont été étudiés selon la méthode décrite par G.L. Ellman et al., Biochem. Pharmacol. 7, 88-95 (1961).

Les résultats obtenus sur certains composés de l'invention sont reportés, à titre d'exemple, dans le tableau 2.

Tableau 2

| Inhibition de l'activité de l'acétylcholinestérase | |
|---|---|
| Composé n° | $IC_{50}$ (nM) |
| 5 | 50 |
| 11 | 26 |
| 12 | 30 |
| 13 | 15 |
| 14 | 55 |
| 16 | 100 |
| Tacrine | 120 |

Les composés de l'invention sont des inhibiteurs de l'acétylcholinestérase ; aussi, ils peuvent être utiles dans le traitement des maladies telles que la myasthénie, les troubles de la mémoire, les démences; telles que les démences séniles ou la maladie d'Alzheimer.

Les préparations pharmaceutiques contenant ces principes actifs peuvent être mises en forme pour l'administration par voie orale, rectale, parentérale ou locale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

**Revendications**

**Revendications pour les Etats contractants suivants : AT, BE, CH, DE, DK, FR, GB, GR, IT, LI, LU, MC, NL, PT, SE**

**1.** Dérivés de l'urée correspondant à la formule générale 1

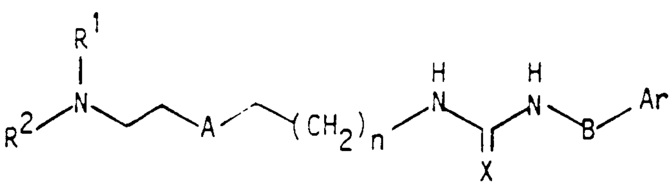

1

dans laquelle :

- $R^1$ représente un groupe alkyle en $C_1$-$C_4$ :
- $R^2$ représente :
  - un groupe cycloalkyle en $C_5$-$C_7$
  - un groupe cycloalkylméthyle où le radical cycloalkyle comporte de 5 à 7 atomes de carbone
  - un groupe benzyle
  - un groupe benzyle où le cycle aromatique porte un groupe alkyle en $C_1$-$C_4$, un groupe alkoxy en $C_1$-$C_4$, un atome d'halogéne ou un groupe nitro ;
- A représente un atome d'oxygène ou un radical méthylène ;
- n représente 1 ou 2 ;
- X représente un atome d'oxygène ou de soufre ;
- B représente une liaison directe, un radical méthylène ou un radical carbonyle ;
- Ar représente :
  - un groupe pyridyle

- un groupe phényle de formule:

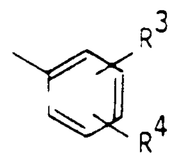

où $R_3$ et $R_4$, indépendamment l'un de l'autre, représentent un atome d'hydrogène, un atome d'halogène, un groupe nitro, un groupe alkyle en $C_1$-$C_4$, un groupe alkoxy en $C_1$-$C_4$, un groupe phényle, ou un groupe trifluorométhoxy ;
- un groupe oxofluorényle de formule :

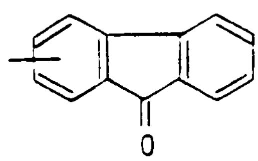

- un groupe dioxoanthracényle de formule :

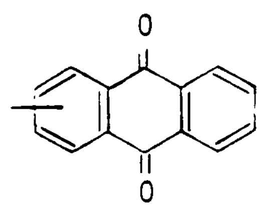

- un groupe naphthyle,
ainsi que les sels organiques ou minéraux thérapeutiquement acceptables de ces molécules.

**2.** Composés de formule générale 1 selon la revendication 1 caractérisés par le fait qu'il sont choisis parmi :
- {[(N-benzyl-N-méthyl)amino-2 éthoxy]-2 éthyl}-1 (nitro-4 phényl)-3 urée
- Benzyl-1 {[(N-cyclohexyl-N-éthyl)amino-2 éthoxy]-2 éthyl}-3 urée
- Benzoyl-1 {[(N-benzyl-N-méthyl)amino-2 éthoxy]-2 éthyl}-3 urée
- Benzoyl-1 [(N-benzyl-N-méthyl)amino-5 pentyl]-3 thiourée
- Benzoyl-1 [(N-cyclohexylméthyl-N-méthyl)amino-5 pentyl]-3 thiourée
- {[(N-benzyl-N-méthyl)amino-2 éthoxy]-2 éthyl}-1 (chloro-4 phényl)-3 thiourée
- {[(N-benzyl-N-méthyl)amino-2 éthoxy]-2 éthyl}-1 (α-naphthyl)-3 thiourée
- Benzoyl-1 {[(N-benzyl-N-méthyl)amino-2 éthoxy]-3 propyl}-3 thiourée
- Benzoyl-1 {[(N-benzyl-N-méthyl)amino-2 éthoxy]-2 éthyl}-3 thiourée
- {[(N-benzyl-N-méthyl)amino-2 éthoxy]-2 éthyl}-1 (méthyl-4 benzoyl)-3 thiourée
- {[(N-benzyl-N-méthyl)amino-2 éthoxy]-2 éthyl}-1 (méthoxy-4 benzoyl)-3 thiourée
- {[(N-benzyl-N-méthyl)amino-2 éthoxy]-2 éthyl}-1 (chloro-4 benzoyl)-3 thiourée
- {[(N-benzyl-N-méthyl)amino-2 éthoxy]-2 éthyl}-1 (nitro-3 benzoyl)-3 thiourée
- {[(N-benzyl-N-méthyl)amino-2 éthoxy]-2 éthyl}-1 (dichloro-3,4 benzoyl)-3 thiourée
- {[(N-benzyl-N-méthyl)amino-2 éthoxy]-2 éthyl}-1 (phényl-4 benzoyl)-3 thiourée
- {[(N-benzyl-N-méthyl)amino-2 éthoxy]-2 éthyl}-1 (β-naphtoyl)-3 thiourée
- {[(N-benzyl-N-méthyl)amino-2 éthoxy]-2 éthyl}-1 [(oxo-9 fluorènoyl)-2]-3 thiourée
- {[(N-benzyl-N-méthyl)amino-2 éthoxy]-2 éthyl}-1 [(dioxo-9,10 anthracénoyl)-2]-3 thiourée
- Benzoyl-1 {[(N-cyclohexyl-N-méthyl)amino-2 éthoxy-2 éthyl}-3 thiourée
- {[(N-méthyl-N-m-nitrobenzyl)amino-2 éthoxy]-2 éthyl}-1 (pyridyl-3)-3 thiourée
- {[(N-benzyl-N-méthyl)amino-2 éthoxy]-2 éthyl}-1 (trifluorométhoxy-4 phényl)-3 thiourée

- (Diméthoxy-3,4 phényl)-1 {[(N-méthyl-N-m-nitrobenzyl)amino-2 éthoxy]-2 éthyl}-3 thiourée

3. procédé de préparation de composés selon les revendications 1 et 2 caractérisé en ce que l'on fait réagir une amine de formule générale 2 avec un composé de formule générale 3 :

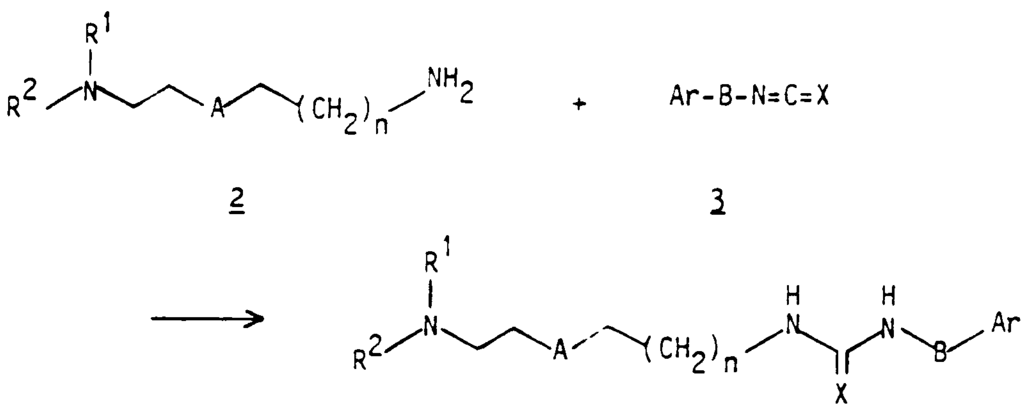

2 3

1

où $R^1$, $R^2$, A, n, X, B et Ar sont définis comme ci-dessus.

4. Procédé de préparation de composés selon la revendication 3 caractérisé en ce que la réaction d'une amine de formule générale 2 avec un isothiocyanate de formule générale 3, où X représente un atome de soufre, s'effectue en solution dans un solvant chloré, par exemple, le dichlorométhane ou le dichloroéthane, un solvant cétonique, par exemple l'acétone, un solvant alcoolique, par exemple l'éthanol, ou un éther tel que le tétrahydrofurane à une température comprise entre la température ambiante et celle du reflux du solvant.

5. Procédé de préparation de composés selon la revendication 3 caractérisé en ce que la réaction d'une amine de formule générale 2 avec un isocyanate de formule générale 3, où X représente un atome d'oxygène, s'effectue en solution, de préférence à une température comprise entre 0 °C et 35 °C, au sein d'un solvant tel que l'acétone, le toluène, un solvant chloré tel que le dichlorométhane ou le dichloroéthane, ou un éther tel que le tétrahydrofurane.

6. A titre de médicament, les dérivés de formule générale 1 selon l'une des revendications 1 ou 2.

7. Médicaments selon la revendication 6, utiles dans le traitement de la myasthénie, les troubles de la mémoire, les démences, telles que les démences séniles ou la maladie d'Alzheimer.

8. Utilisation d'un composé de formule générale 1 selon l'une des revendications 1 ou 2, pour la préparation d'un médicament destiné au traitement de la myasthénie, les troubles de la mémoire, les démences, telles que les démences séniles ou la maladie d'Alzheimer.

9. Composition pharmaceutique caractérisée en ce qu'elle contient un composé défini selon l'une des revendications 1 ou 2.

**Revendications pour l'Etat contractant suivant : ES**

**1.** Procédé de préparation de dérivés de l'urée correspondant à la formule générale 1

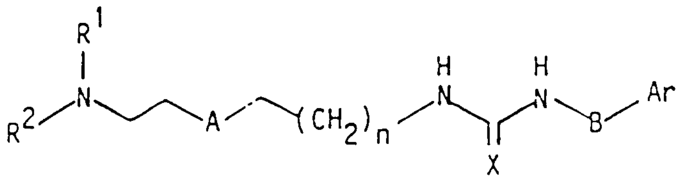

1

dans laquelle :

R1 représente un groupe alkyle en $C_1$-$C_4$ ;

R2 représente :

- un groupe cycloalkyle en $C_5$-$C_7$ ;
- un groupe cycloalkylméthyle où le radical cycloalkyle comporte de 5 à 7 atomes de carbone,
- un groupe benzyle,
- un groupe benzyle où le cycle aromatique porte un groupe alkyle en $C_1$-$C_4$, un groupe alkoxy en $C_1$-$C_4$, un atome d'halogène ou un groupe nitro:

A représente un atome d'oxygène ou un radical méthylène;

n représente 1 ou 2 ;

X représente un atome d'oxygène ou de soufre ;

B représente une laison directe, un radical méthlène ou un radical carbonyle ;

Ar représente :

- un groupe pyridyle
- un groupe phényle de formule :

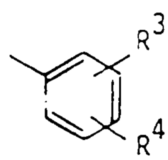

où R3 et R4, indépendamment l'un de l'autre,
représentent un atome d'hydrogène, un atome d'halogène, un groupe nitro, un groupe alkyle en $C_1$-$C_4$, un groupe alkoxy en $C_1$-$C_4$, un groupe phényle, ou un groupe trifluorométhoxy ;

- un groupe oxofluorényle de formule :

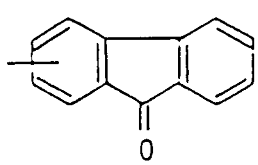

- un groupe dioxoanthracényle de formule :

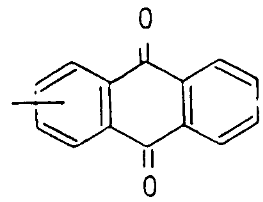

- un groupe naphtyle,
ainsi que les sels organiques ou minéraux thérapeutiquement acceptables de ces molécules.

caractérisé en ce que l'on fait réagir une amine de formule générale 2 avec un composé de formule générale 3 :

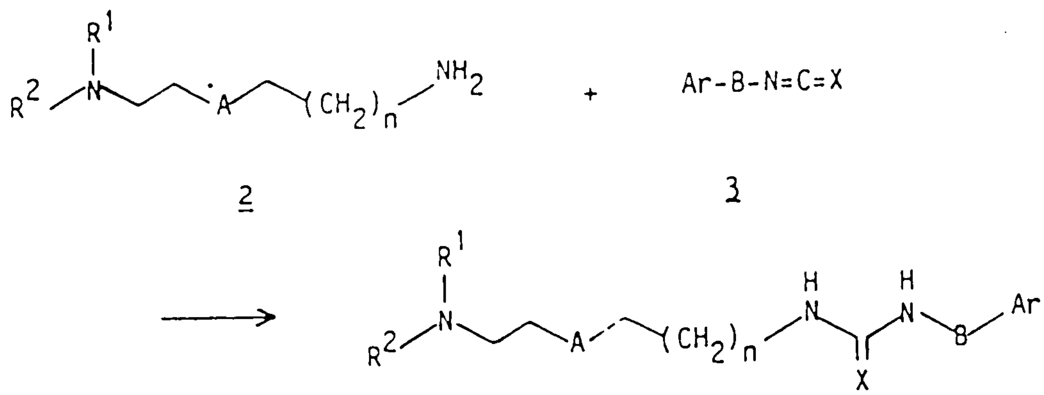

2

3

1

où R1, R2, A, n, X, B et Ar sont définis comme ci-dessus.

**2.** Procédé selon la revendication 1, caractérisé en ce que X représente un atome de soufre, la réaction d'une amine de formule générale 2 avec un composé de formule générale 3 étant effectuée à une température allant de la température ambiante à la température de reflux du solvant, le solvant employé étant choisi parmi un solvant chloré tel que le dichlorométhane ou le dichloroéthane, un solvant cétonique tel que l'acétone, un solvant alcoolique, tel que l'éthanol ou un éther tel que le tétrahydrofurane.

**3.** Procédé selon la revendication 1, caractérisé en ce que X représente un atome d'oxygène, la réaction entre une amine de formule générale 2 et un isocyanate de formule générale 3 étant effectuée à une température comprise entre 0°C et 35°C au sein d'un solvant aprotique, choisi parmi l'acétone, le toluène, un solvant chloré tel que le dichlorométhane ou le dichloroéthane, ou un éther tel que le tétrahydrofurane.

**4.** Procédé selon la revendication 1, caractérisé en ce que les dérivés de formule générale 1 sont choisis parmi :
- {[(N-benzyl-N-méthyl)amino-2 éthoxy]-2 éthyl}-1 (nitro-4 phényl)-3 urée
- Benzyl-1 {[(N-cyclohexyl-N-éthyl)amino-2 éthoxy]-2 éthyl}-3 urée
- Benzoyl-1 {[(N-benzyl-N-méthyl)amino-2 éthoxy]-2 éthyl}-3 urée
- Benzoyl-1 [(N-benzyl-N-méthyl)amino-5 pentyl]-3 thiourée
- Benzoyl-1 [(N-cyclohexylméthyl-N-méthyl)amino-5 pentyl]-3 thiourée
- {[(N-benzyl-N-méthyl)amino-2 éthoxy]-2 éthyl}-1 (chloro-4 phényl)-3 thiourée
- {[(N-benzyl-N-méthyl)amino-2 éthoxy]-2 éthyl)-1 (α-naphthyl)-3 thiourée
- Benzoyl-1 {[(N-benzyl-N-méthyl)amino-2 éthoxy]-3 propyl}-3 thiourée
- Benzoyl-1 {[(N-benzyl-N-méthyl)amino-2 éthoxy]-2 éthyl}-3 thiourée

- {[(N-benzyl-N-méthyl)amino-2 éthoxy]-2 éthyl}-1 (méthyl-4 benzoyl)-3 thiourée
- {[(N-benzyl-N-méthyl)amino-2 éthoxy]-2 éthyl}-1 (méthoxy-4 benzoyl)-3 thiourée
- {[(N-benzyl-N-méthyl)amino-2 éthoxy]-2 éthyl}-1 (chloro-4 benzoyl)-3 thiourée
- {[(N-benzyl-N-méthyl)amino-2 éthoxy]-2 éthyl}-1 (nitro-3 benzoyl)-3 thiourée
- {[(N-benzyl-N-méthyl)amino-2 éthoxy]-2 éthyl}-1 (dichloro-3,4 benzoyl)-3 thiourée
- {[(N-benzyl-N-méthyl)amino-2 éthoxy]-2 éthyl}-1 (phényl-4 benzoyl)-3 thiourée
- {[(N-benzyl-N-méthyl)amino-2 éthoxy]-2 éthyl}-1 ($\beta$-naphtoyl)-3 thiourée
- {[(N-benzyl-N-méthyl)amino-2 éthoxy]-2 éthyl}-1 [(oxo-9 fluorènoyl)-2]-3 thiourée
- {[(N-benzyl-N-méthyl)amino-2 éthoxy]-2 éthyl}-1 [(dioxo-9,10 anthracénoyl)-2]-3 thiourée
- Benzoyl-1 {[(N-cyclohexyl-N-méthyl)amino-2 éthoxy-2 éthyl}-3 thiourée
- {[(N-méthyl-N-m-nitrobenzyl)amino-2 éthoxy]-2 éthyl}-1 (pyridyl-3)-3 thiourée
- {[(N-benzyl-N-méthyl)amino-2 éthoxy]-2 éthyl}-1 (trifluorométhoxy-4 phényl)-3 thiourée
- (Diméthoxy-3,4 phényl)-1 {[(N-méthyl-N-m-nitrobenzyl)amino-2 éthoxy]-2 éthyl}-3 thiourée

## Claims

### Claims for the following Contracting States : AT, BE, CH, DE, DK, FR, GB, GR, IT, LI, LU, MC, NL, PT, SE

**1.** Urea derivatives corresponding to the general formula 1

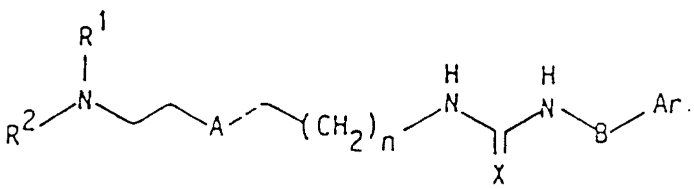

1

in which:

- $R^1$ represents a $C_1$-$C_4$ alkyl group;
- $R^2$ represents:
  - a $C_5$-$C_7$ cycloalkyl group
  - a cycloalkylmethyl group in which the cycloalkyl radical contains from 5 to 7 carbon atoms
  - a benzyl group
  - a benzyl group in which the aromatic ring bears a $C_1$-$C_4$ alkyl group, a $C_1$-$C_4$ alkoxy group, a halogen atom or a nitro group;
- A represents an oxygen atom or a methylene radical;
- n represents 1 or 2;
- X represents an oxygen or sulphur atom;
- B represents a direct bond, a methylene radical or a carbonyl radical;
- Ar represents:
  - a pyridyl group
  - a phenyl group of formula:

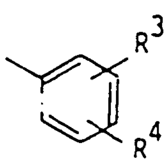

where $R^3$ and $R^4$, independently of one another, represent a hydrogen atom, a halogen atom, a nitro group, a $C_1$-$C_4$ alkyl group, a $C_1$-$C_4$ alkoxy group, a phenyl group or a

trifluoromethoxy group;

- an oxofluorenyl group of formula:

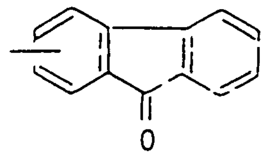

- a dioxoanthracenyl group of formula:

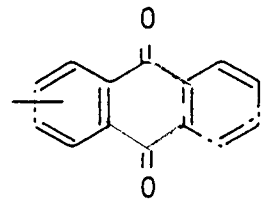

- a naphthyl group,

as well as the therapeutically acceptable organic or inorganic salts of these molecules.

**2.** Compounds of general formula 1 according to Claim 1, characterized in that they are chosen from:

- 1-{2-[2-(N-Benzyl-N-methylamino)ethoxy]ethyl}-3-(4-nitrophenyl)urea
- 1-Benzyl-3-{2-[2-(N-cyclohexyl-N-ethylamino)ethoxy]ethyl}urea
- 1-Benzoyl-3-{2-[2-(N-benzyl-N-methylamino)ethoxy]ethyl}urea
- 1-Benzoyl-3-[5-(N-benzyl-N-methylamino)pentyl]thiourea
- 1-Benzoyl-3-[5-(N-cyclohexylmethyl-N-methylamino)pentyl]thiourea
- 1-{2-[2-(N-Benzyl-N-methylamino)ethoxy]ethyl}-3-(4-chlorophenyl)thiourea
- 1-{2-[2-(N-Benzyl-N-methylamino)ethoxy]ethyl}-3-(α-naphthyl)thiourea
- 1-Benzoyl-3-{3-[2-(N-benzyl-N-methylamino)ethoxy]propyl}thiourea
- 1-Benzoyl-3-{2-[2-(N-benzyl-N-methylamino)ethoxy]ethyl}thiourea
- 1-{2-[2-(N-Benzyl-N-methylamino)ethoxy]ethyl}-3-(4-methylbenzoyl)thiourea
- 1-{2-[2-(N-Benzyl-N-methylamino)ethoxy]ethyl}-3-(4-methoxybenzoyl)thiourea
- 1-{2-[2-(N-Benzyl-N-methylamino)ethoxy]ethyl}-3-(4-chlorobenzoyl)thiourea
- 1-{2-[2-(N-Benzyl-N-methylamino)ethoxy]ethyl}-3-(3-nitrobenzoyl)thiourea
- 1-{2-[2-(N-Benzyl-N-methylamino)ethoxy]ethyl}-3-(3,4-dichlorobenzoyl)thiourea
- 1-{2-[2-(N-Benzyl-N-methylamino)ethoxy)ethyl}-3-(4-phenylbenzoyl)thiourea
- 1-{2-[2-(N-Benzyl-N-methylamino)ethoxy]ethyl}-3-(β-naphthoyl)thiourea
- 1-{2-[2-(N-Benzyl-N-methylamino)ethoxy]ethyl}-3-(9-oxo-2-fluorenoyl)thiourea
- 1-{2-[2-(N-Benzyl-N-methylamino)ethoxy]ethyl}-3-(9,10-dioxo-2-anthracenoyl)thiourea
- 1-Benzoyl-3-{2-[2-(N-cyclohexyl-N-methylamino)ethoxy]ethyl}thiourea
- 1-[2-{2-[N-Methyl-N-(m-nitrobenzyl)amino]ethoxy}ethyl]-3-(3-pyridyl)thiourea
- 1-{2-[2-(N-Benzyl-N-methylamino)ethoxy]ethyl}-3-(4-trifluoromethoxyphenyl)thiourea
- 1-(3,4-Dimethoxyphenyl)-3-[2-{2-[N-methyl-N-(m-nitrobenzyl)amino]ethoxy}ethyl]thiourea

**3.** Process for preparing compounds according to Claims 1 and 2, characterized in that an amine of general formula 2 is reacted with a compound of general formula 3:

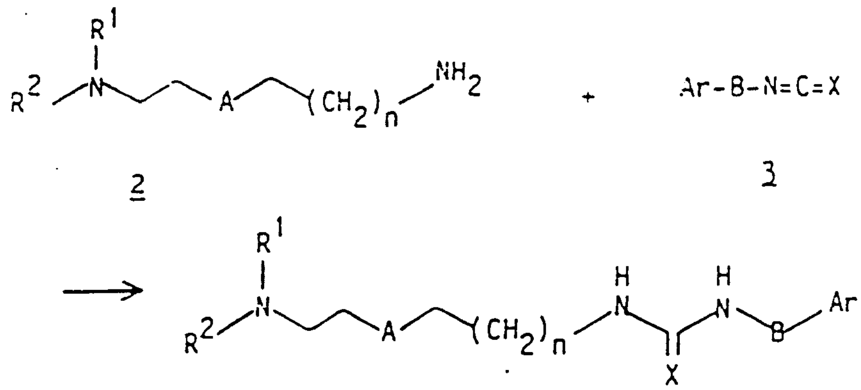

2 3

1

where $R^1$, $R^2$, A, n, X, B and Ar are defined as above.

4. Process for preparing compounds according to Claim 3, characterized in that the reaction of an amine of general formula 2 with an isothiocyanate of general formula 3, where X represents a sulphur atom, is performed in solution in a chlorinated solvent, for example dichloromethane or dichloroethane, a ketonic solvent, for example acetone, an alcoholic solvent, for example ethanol, or an ether such as tetrahydrofuran, at a temperature between room temperature and the refluxing temperature of the solvent.

5. Process for preparing compounds according to Claim 3, characterized in that the reaction of an amine of general formula 2 with an isocyanate of general formula 3, where X represents an oxygen atom, is performed in solution, preferably at a temperature of between 0°C and 35°C, in a solvent such as acetone, toluene, a chlorinated solvent such as dichloromethane or dichloroethane or an ether such as tetrahydrofuran.

6. As a medicinal product, the derivatives of general formula 1 according to one of Claims 1 or 2.

7. Medicinal products according to Claim 6, which are useful in the treatment of myasthenia, memory disorders, dementia such as senile dementia or Alzheimer's disease.

8. Use of a compound of general formula 1 according to one of Claims 1 and 2, for preparing a medicinal product intended for the treatment of myasthenia, memory disorders, dementia such as senile dementia or Alzheimer's disease.

9. Pharmaceutical composition, characterized in that it contains a compound defined according to one of Claims 1 and 2.

**Claims for the following Contracting State : ES**

1. Process for preparing urea derivatives corresponding to the general formula 1

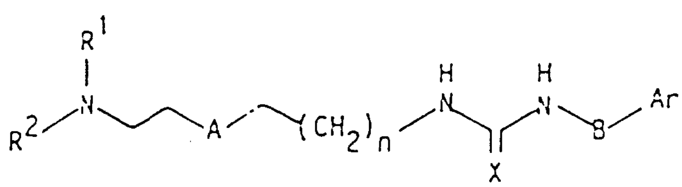

1

in which:

$R^1$ represents a $C_1$-$C_4$ alkyl group;

$R^2$ represents:

- a $C_5$-$C_7$ cycloalkyl group
- a cycloalkylmethyl group in which the cyclo alkyl radical contains from 5 to 7 carbon atoms
- a benzyl group
- a benzyl group in which the aromatic ring bears a $C_1$-$C_4$ alkyl group, a $C_1$-$C_4$ alkoxy group, a halogen atom or a nitro group;

A represents an oxygen atom or a methylene radical;

n represents 1 or 2;

X represents an oxygen or sulphur atom;

B represents a direct bond, a methylene radical or a carbonyl radical;

Ar represents:

- a pyridyl group
- a phenyl group of formula:

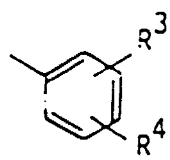

where $R^3$ and $R^4$, independently of one another, represent a hydrogen atom, a halogen atom, a nitro group, a $C_1$-$C_4$ alkyl group, a $C_1$-$C_4$ alkoxy group, a phenyl group or a trifluoromethoxy group;

- an oxofluorenyl group of formula:

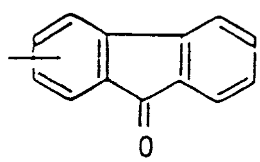

- a dioxoanthracenyl group of formula:

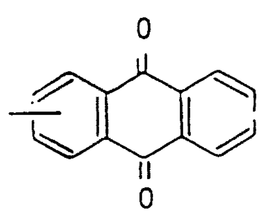

- a naphthyl group,

as well as the therapeutically acceptable organic or inorganic salts of these molecules, characterized in that an amine of general formula 2 is reacted with a compound of general formula 3:

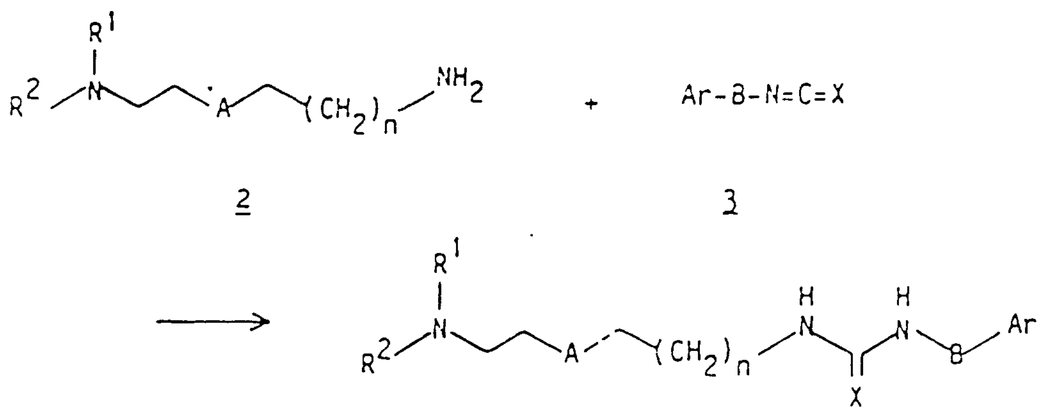

2 3

1

where $R^1$, $R^2$, A, n, X, B and Ar are defined as above.

2. Process according to Claim 1, characterized in that X represents a sulphur atom, the reaction of an amine of general formula 2 with a compound of general formula 3 being performed at a temperature ranging from room temperature to the refluxing temperature of the solvent,
the solvent employed being chosen from a chlorinated solvent such as dichloromethane or dichloroethane, a ketonic solvent such as acetone, an alcoholic solvent such as ethanol or an ether such as tetrahydrofuran.

3. Process according to Claim 1, characterized in that X represents an oxygen atom, the reaction between an amine of general formula 2 and an isocyanate of general formula 3 being performed at a temperature of between 0°C and 35°C, in an aprotic solvent chosen from acetone, toluene, a chlorinated solvent such as dichloromethane or dichloroethane or an ether such as tetrahydrofuran.

4. Process according to Claim 1, characterized in that the derivatives of general formula 1 are chosen from:
- 1-{2-[2-(N-Benzyl-N-methylamino)ethoxy]ethyl}-3-(4-nitrophenyl)urea
- 1-Benzyl-3-{2-[2-(N-cyclohexyl-N-ethylamino)ethoxy]ethyl}urea
- 1-Benzoyl-3-{2-[2-(N-benzyl-N-methylamino)ethoxy]ethyl}urea
- 1-Benzoyl-3-[5-(N-benzyl-N-methylamino)pentyl]thiourea
- 1-Benzoyl-3-[5-(N-cyclohexylmethyl-N-methylamino)pentyl]thiourea
- 1-{2-[2-(N-Benzyl-N-methylamino)ethoxy]ethyl}-3-(4-chlorophenyl)thiourea
- 1-{2-[2-(N-Benzyl-N-methylamino)ethoxy]ethyl}-3-(α-naphthyl)thiourea
- 1-Benzoyl-3-{3-[2-(N-benzyl-N-methylamino)ethoxy]propyl}thiourea
- 1-Benzoyl-3-{2-[2-(N-benzyl-N-methylamino)ethoxy]ethyl}thiourea
- 1-{2-[2-(N-Benzyl-N-methylamino)ethoxy]ethyl}-3-(4-methylbenzoyl)thiourea
- 1-{2-[2-(N-Benzyl-N-methylamino)ethoxy]ethyl}-3-(4-methoxybenzoyl)thiourea
- 1-{2-[2-(N-Benzyl-N-methylamino)ethoxy]ethyl}-3-(4-chlorobenzoyl)thiourea
- 1-{2-[2-(N-Benzyl-N-methylamino)ethoxy]ethyl}-3-(3-nitrobenzoyl)thiourea
- 1-{2-[2-(N-Benzyl-N-methylamino)ethoxy]ethyl}-3-(3,4-dichlorobenzoyl)thiourea
- 1-{2-[2-(N-Benzyl-N-methylamino)ethoxy]ethyl}-3-(4-phenylbenzoyl)thiourea
- 1-{2-[2-(N-Benzyl-N-methylamino)ethoxy]ethyl}-3-(β-naphthoyl)thiourea
- 1-{2-[2-(N-Benzyl-N-methylamino)ethoxy]ethyl}-3-(9-oxo-2-fluorenoyl)thiourea
- 1-{2-[2-(N-Benzyl-N-methylamino)ethoxy]ethyl}-3-(9,10-dioxo-2-anthracenoyl)thiourea
- 1-Benzoyl-3-{2-[2-(N-cyclohexyl-N-methylamino)ethoxy]ethyl}thiourea
- 1-[2-{2-[N-Methyl-N-(m-nitrobenzyl)amino]ethoxy}ethyl]-3-(3-pyridyl)thiourea
- 1-{2-[2-(N-Benzyl-N-methylamino)ethoxy]ethyl}-3-(4-trifluoromethoxyphenyl)thiourea
- 1-(3,4-Dimethoxyphenyl)-3-[2-{2-[N-methyl-N-(m-nitrobenzyl)amino]ethoxy}ethyl]thiourea.

## Patentansprüche

## Patentansprüche für folgende Vertragsstaaten : AT, BE, CH, DE, DK, FR, GB, GR, IT, LI, LU, MC, NL, PT, SE

**1.** Harnstoffderivate der allgemeinen Formel (1)

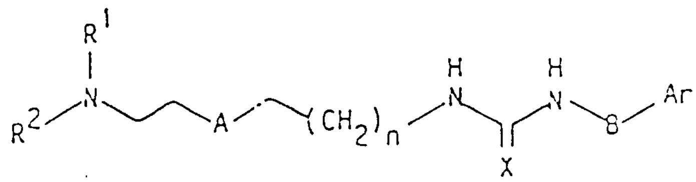

1

worin bedeuten:

$R^1$ eine $C_1$-$C_4$-Alkylgruppe;

$R^2$

- eine $C_5$-$C_7$-Cycloalkylgruppe,
- eine Cycloalkylmethylgruppe, in der der Cycloalkylrest 5 bis 7 Kohlenstoffatome enthält,
- eine Benzylgruppe,
- eine Benzylgruppe, in der der aromatische Ring eine $C_1$-$C_4$-Alkylgruppe, eine $C_1$-$C_4$-Alkoxygruppe, ein Halogenatom oder eine Nitrogruppe trägt;

A eine Sauerstoffatom oder einen Methylenrest;

n die Zahl 1 oder 2;

X ein Sauerstoffatom oder ein Schwefelatom;

B eine direkte Bindung, einen Methylenrest oder einen Carbonylrest;

Ar

- eine Pyridylgruppe,
- eine Phenylgruppe der Formel

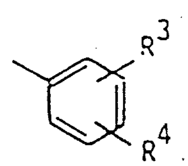

worin $R^3$ und $R^4$ unabhängig voneinander für ein Wasserstoffatom, ein Halogenatom, eine Nitrogruppe, eine $C_1$-$C_4$-Alkylgruppe, eine $C_1$-$C_4$-Alkoxygruppe, eine Phenylgruppe oder eine Trifluoromethoxygruppe stehen;

- eine Oxofluorenylgruppe der Formel

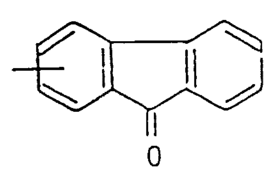

- eine Dioxoanthracenylgruppe der Formel

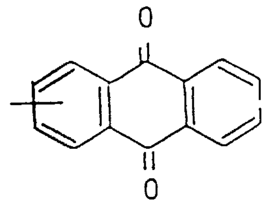

- eine Naphthylgruppe,
sowie die therapeutisch akzeptablen organischen oder Mineralsalze dieser Moleküle.

**2.** Verbindungen der allgemeinen Formel (1) nach Anspruch 1, dadurch gekennzeichnet, daß sie ausgewählt werden aus der Gruppe:
- 1-{2-[2-(N-Benzyl-N-methyl)amino-ethoxy]ethyl}-3-(4-nitrophenyl)-harnstoff,
- 1-Benzyl-3-{2-[2-(N-cyclohexyl-N-ethyl)amino-ethoxy]ethyl}-harnstoff,
- 1-Benzoyl-3-{2-[2-(N-benzyl-N-methyl)amino-ethoxy]ethyl}-harnstoff,
- 1-Benzoyl-3-[5-(N-benzyl-N-methyl)amino-pentyl]-thioharnstoff,
- 1-Benzoyl-3-[5-(N-cyclohexylmethyl-N-methyl)aminopentyl]thioharnstoff,
- 1-{2-[2-(N-Benzyl-N-methyl)amino-ethoxy]-ethyl}-3-(4-chlorophenyl)-thioharnstoff,
- 1-{2-[2-(N-Benzyl-N-methyl)amino-ethoxy]-ethyl}-3-(α-naphthyl)-thioharnstoff,
- 1-Benzoyl-3-{3-[2-(N-benzyl-N-methyl)amino-ethoxy]-propyl}-thioharnstoff,
- 1-Benzoyl-3-{2-[2-(N-benzyl-N-methyl)amino-ethoxy]-ethyl}-thioharnstoff,
- 1-{2-[2-(N-Benzyl-N-methyl)amino-ethoxy]-ethyl}-3-(4-methylbenzoyl)-thioharnstoff,
- 1-{2-[2-(N-Benzyl-N-methyl)amino-ethoxy]-ethyl}-3-(4-methoxybenzoyl)-thioharnstoff
- 1-{2-[2-(N-Benzyl-N-methyl)amino-ethoxy]-ethyl}-3-(4-chlorobenzoyl)-thioharnstoff,
- 1-{2-[2-(N-Benzyl-N-methyl)amino-ethoxy]-ethyl}-3-(3-nitrobenzoyl)-thioharnstoff,
- 1-{2-[2-(N-Benzyl-N-methyl)amino-ethoxy]-ethyl}-3-(3,4-dichlorobenzoyl)-thioharnstoff,
- 1-{2-[2-(N-Benzyl-N-methyl)amino-ethoxy]-ethyl}-3-(4-phenylbenzoyl)-thioharnstoff,
- 1-{2-[2-(N-Benzyl-N-methyl)amino-ethoxy]-ethyl}-3-(β-naphthoyl)-thioharnstoff,
- 1-{2-[2-(N-Benzyl-N-methyl)amino-ethoxy]-ethyl}-3-[2-(9-oxofluorenoyl)]-thioharnstoff,
- 1-{2-[2-(N-Benzyl-N-methyl)amino-ethoxy]-ethyl}-3-[2-(9,10-dioxoanthracenoyl)]-thioharnstoff,
- 1-Benzoyl-3-{2-[2-(N-cyclohexyl-N-methyl)amino-ethoxy]-ethyl}-thioharnstoff,
- 1-{2-[2-(N-Methyl-N-m-nitrobenzyl)amino-ethoxy]-ethyl}-3-(3-pyridyl)-thioharnstoff,
- 1-{2-[2-(N-Benzyl-N-methyl)amino-ethoxy]-ethyl}-(3-(4-trifluoromethoxyphenyl)-thioharnstoff,
- 1-(3,4-Dimethoxyphenyl)-3-{2-[2-(N-methyl-N-m-nitrobenzyl)amino-ethoxy]-ethyl}-thioharnstoff.

**3.** Verfahren zur Herstellung der Verbindungen nach den Ansprüchen 1 und 2, dadurch gekennzeichnet, daß man ein Amin der allgemeinen Formel (2) mit einer Verbindung der allgemeinen Formel (3) reagieren läßt:

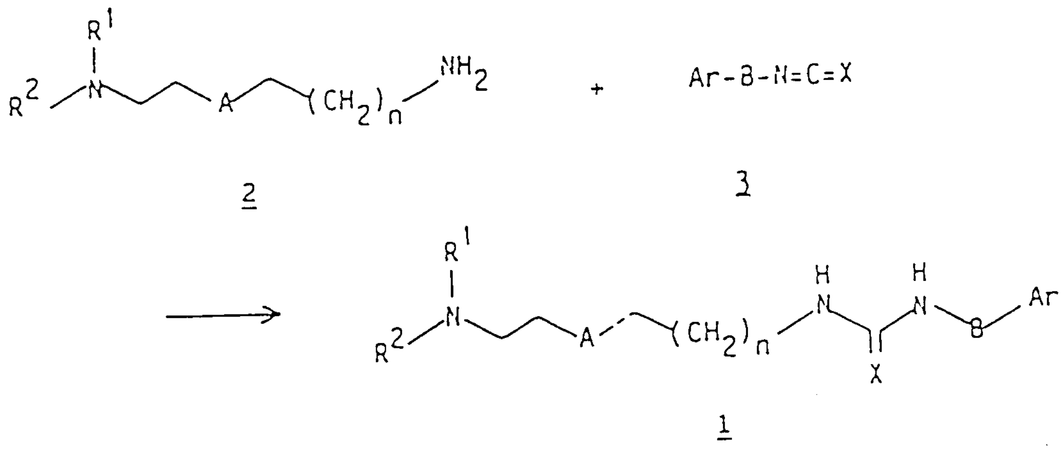

worin $R^1$, $R^2$, A, n, X, B und Ar wie oben definiert sind.

**4.** Verfahren zur Herstellung von Verbindungen nach Anspruch 3, dadurch gekennzeichnet, daß man ein Amin der allgemeinen Formel (2) mit einem Isothiocyanat der allgemeinen Formel (3), worin X ein Schwefelatom darstellt, gelöst in einem chlorierten Lösungsmittel wie Dichlormethan oder Dichlorethan, in einem Ketonlösungsmittel, wie Aceton, in einem alkoholischen Lösungsmittel wie Ethanol oder in einem Äther, wie Tetrahydrofuran, bei einer Temperatur zwischen Umgebungstemperatur und der Rückflußtemperatur des Lösungsmittels reagieren läßt.

**5.** Verfahren zur Herstellung der Verbindungen nach Anspruch 3, dadurch gekennzeichnet, daß man ein Amin der allgemeinen Formel (2) mit einem Isocyanat der allgemeinen Formel (3), worin X ein Sauerstoffatom darstellt, in Lösung vorzugsweise bei einer Temperatur zwischen 0 und 35 °C in einem Lösungsmittelmedium, wie Aceton, Toluol, in einem chlorierten Lösungsmittel wie Dichlormethan oder Dichlorethan oder in einem Äther wie Tetrahydrofuran umsetzt.

**6.** Die Derivate der allgemeinen Formel (1) nach einem der Ansprüche 1 oder 2 als Arzneimittel.

**7.** Arzneimittel nach Anspruch 6, die verwendbar sind für die Behandlung der Myasthenie, von Gedächtnisstörungen, Dementia, wie Altersdementia oder der Alzheimer-Krankheit.

**8.** Verwendung einer Verbindung der allgemeinen Formel (1) nach einem der Ansprüche 1 oder 2 zur Herstellung eines Arzneimittels für die Behandlung der Myasthenie, von Gedächtnisstörungen, von Dementia, wie Altersdementia oder der Alzheimer-Krankheit.

**9.** Pharmazeutische Zusammensetzung, dadurch gekennzeichnet, daß sie eine Verbindung nach einem der Ansprüche 1 oder 2 enthält.

**Patentansprüche für folgenden Vertragsstaat : ES**

**1.** Verfahren zur Herstellung von Harnstoffderivaten der allgemeinen Formel (1)

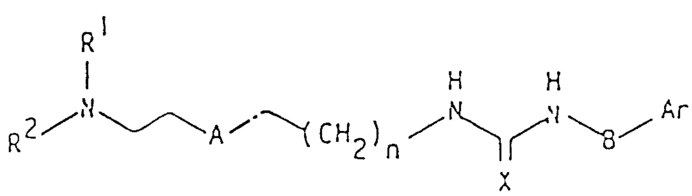

1

worin bedeuten:

$R^1$ eine $C_1$-$C_4$-Alkylgruppe;

$R^2$

- eine $C_5$-$C_7$-Cycloalkylgruppe,
- eine Cycloalkylmethylgruppe, in der der Cycloalkylrest 5 bis 7 Kohlenstoffatome enthält,
- eine Benzylgruppe,
- eine Benzylgruppe, in der der aromatische Ring eine $C_1$-$C_4$-Alkylgruppe, eine $C_1$-$C_4$-Alkoxygruppe, ein Halogenatom oder eine Nitrogruppe trägt;

A eine Sauerstoffatom oder einen Methylenrest;

n die Zahl 1 oder 2;

X ein Sauerstoffatom oder ein Schwefelatom;

B eine direkte Bindung, einen Methylenrest oder einen Carbonylrest;

Ar

- eine Pyridylgruppe,

- eine Phenylgruppe der Formel

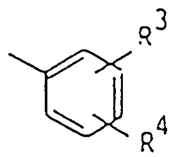

worin $R^3$ und $R^4$ unabhängig voneinander für ein Wasserstoffatom, ein Halogenatom, eine Nitrogruppe, eine $C_1$-$C_4$-Alkylgruppe, eine $C_1$-$C_4$-Alkoxygruppe, eine Phenylgruppe oder eine Trifluoromethoxygruppe stehen;
- eine Oxofluorenylgruppe der Formel

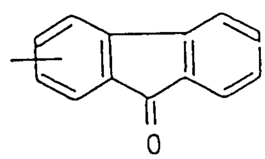

- eine Dioxoanthracenylgruppe der Formel

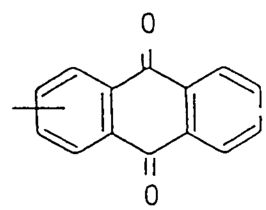

- eine Naphthylgruppe,

sowie die therapeutisch akzeptablen organischen oder Mineralsalze dieser Moleküle, dadurch gekennzeichnet, daß man ein Amin der allgemeinen Formel (2) mit einer Verbindung der allgemeinen Formel (3) reagieren läßt:

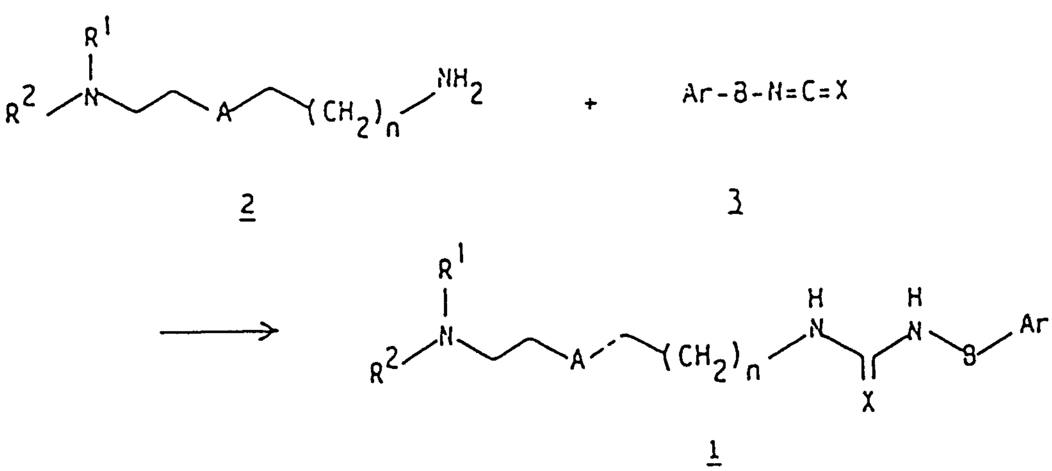

2

3

1

worin $R^1$, $R^2$, A, n, X, B und Ar wie oben definiert sind.

**2.** Verfahren nach Anspruch 1 dadurch gekennzeichnet, daß X ein Schwefelatom darstellt, daß die Umsetzung zwischen einem Amin der allgemeinen Formel (2) und einer Verbindung der allgemeinen Formel (3) bei einer Temperatur von Umgebungstemperatur bis zur Rückflußtemperatur des Lösungsmittels durchgeführt wird, daß das verwendete Lösungsmittel ausgewählt wird aus einem chlorierten Lösungsmittel wie Dichlormethan oder Dichlorethan, einem Ketonlösungsmittel wie Aceton, einem

alkoholischen Lösungsmittel wie Ethanol oder in einem Äther wie Tetrahydrofuran.

**3.** Verfahren nach Anspruch 1, dadurch gekennzeichnet, daß X ein Sauerstoffatom darstellt, daß die Umsetzung zwischen einem Amin der allgemeinen Formel (2) und einem Isocyanat der allgemeinen Formel (3) bei einer Temperatur zwischen 0 und 35°C in einem aprotischen Lösungsmittelmedium, ausgewählt aus Aceton, Toluol, einem chlorierten Lösungsmittel wie Dichlormethan oder Dichlorethan oder einem Äther wie Tetrahydrofuran durchgeführt wird.

**4.** Verfahren nach Anspruch 1, dadurch gekennzeichnet, daß die Derivate der allgemeinen Formel (1) ausgewählt werden aus der Gruppe:

- 1-{2-[2-(N-Benzyl-N-methyl)amino-ethoxy]ethyl}-3-(4-nitrophenyl)-harnstoff,
- 1-Benzyl-3-{2-[2-(N-cyclohexyl-N-ethyl)amino-ethoxy]ethyl}-harnstoff,
- 1-Benzoyl-3-{2-[2-(N-benzyl-N-methyl)amino-ethoxy]ethyl}-harnstoff,
- 1-Benzoyl-3-[5-(N-benzyl-N-methyl)amino-pentyl]-thioharnstoff,
- 1-Benzoyl-3-[5-(N-cyclohexylmethyl-N-methyl)aminopentyl]thioharnstoff,
- 1-{2-[2-(N-Benzyl-N-methyl)amino-ethoxy]-ethyl}-3-(4-chlorophenyl)-thioharnstoff,
- 1-{2-[2-(N-Benzyl-N-methyl)amino-ethoxy]-ethyl}-3-(α-naphthyl)-thioharnstoff,
- 1-Benzoyl-3-{3-[2-(N-benzyl-N-methyl)amino-ethoxy]-propyl}-thioharnstoff,
- 1-Benzoyl-3-{2-[2-(N-benzyl-N-methyl)amino-ethoxy]-ethyl}-thioharnstoff,
- 1-{2-[2-(N-Benzyl-N-methyl)amino-ethoxy]-ethyl}-3-(4-methylbenzoyl)-thioharnstoff,
- 1-{2-[2-(N-Benzyl-N-methyl)amino-ethoxy]-ethyl}-3-(4-methoxybenzoyl)-thioharnstoff,
- 1-{2-[2-(N-Benzyl-N-methyl)amino-ethoxy]-ethyl}-3-(4-chlorobenzoyl)-thioharnstoff,
- 1-{2-[2-(N-Benzyl-N-methyl)amino-ethoxy]-ethyl}-3-(3-nitrobenzoyl)-thioharnstoff,
- 1-{2-[2-(N-Benzyl-N-methyl)amino-ethoxy]-ethyl}-3-(3,4-dichlorobenzoyl)-thioharnstoff,
- 1-{2-[2-(N-Benzyl-N-methyl)amino-ethoxy]-ethyl}-3-(4-phenylbenzoyl)-thioharnstoff,
- 1-{2-[2-(N-Benzyl-N-methyl)amino-ethoxy]-ethyl}-3-(β-naphthoyl)-thioharnstoff,
- 1-{2-[2-(N-Benzyl-N-methyl)amino-ethoxy]-ethyl}-3-[2-(9-oxofluorenoyl)]-thioharnstoff,
- 1-{2-[2-(N-Benzyl-N-methyl)amino-ethoxy]-ethyl}-3-[2-(9,10-dioxoanthracenoyl)]-thioharnstoff,
- 1-Benzoyl-3-{2-[2-(N-cyclohexyl-N-methyl)amino-ethoxy]-ethyl}-thioharnstoff,
- 1-{2-[2-(N-Methyl-N-m-nitrobenzyl)amino-ethoxy]-ethyl}-3-(3-pyridyl)-thioharnstoff,
- 1-{2-[2-(N-Benzyl-N-methyl)amino-ethoxy]-ethyl}-(3-(4-trifluoromethoxyphenyl)-thioharnstoff,
- 1-(3,4-Dimethoxyphenyl)-3-{2-[2-(N-methyl-N-m-nitrobenzyl)amino-ethoxy]-ethyl}-thioharnstoff.